# EUROPEAN PATENT APPLICATION

(11) **EP 2 168 502 A2**
(43) Date of publication of application: **31.03.2010**
(21) Application number: 09252310.9
(22) Date of filing: 29.09.2009
(51) Int. Cl.: A61B 17/00, A61B 17/34

(54) **Methods and devices for performing gastroplasties using a multiple port access device**

(30) Priority: 30.09.2008 US 242381
(71) Applicant: Ethicon Endo-Surgery, Inc., Cincinnati, OH 45242 (US)
(72) Inventor: Murray, Michael A., Bellevue Kentucky 41073 (US); Gill, Robert P., Mason Ohio 45040 (US); Hess, Christopher J., Cincinnati Ohio 45206 (US); Voegele, James Walden, Cincinnati Ohio 45249 (US); Weisenburgh, William Bruce, II, Maineville Ohio 45039 (US); Powell, Darrell M., Cincinnati Ohio 45249 (US); Myers, Stephan Ray, Columbus Ohio 43235 (US)
(74) Representative: Tunstall, Christopher Stephen

(57) **Abstract**

Methods and devices are provided for performing gastroplasties. In one embodiment, a method of performing a gastroplasty includes gaining access to a stomach of a patient through an opening formed in the patient's abdominal wall. A multiple port access device having two or more sealing ports through which surgical instruments can be inserted can be positioned in the abdominal opening. Various instruments can be inserted through the various sealing ports to perform certain steps, such as tensioning and cutting tissue, sizing and transecting the stomach, viewing the surgical site, etc. The methods and devices can be used to perform a Magenstrasse and Mill procedure in which only a portion of the stomach is transected.

## Description

### FIELD OF THE INVENTION

The present invention relates to gastroplasties and methods and devices for performing gastroplasties.

### BACKGROUND OF THE INVENTION

Obesity is becoming a growing concern, particularly in the United States, as the number of obese people continues to increase and more is learned about the negative health effects of obesity. Morbid obesity, in which a person is 100 pounds or more over ideal body weight, in particular poses significant risks for severe health problems. Accordingly, a great deal of attention is being focused on treating obese patients. Surgical procedures to treat morbid obesity have included gastric bypasses (stomach stapling), adjustable gastric banding, and vertical banded gastroplasty and sleeve gastrectomies (removal of all or a portion of the stomach). Such surgical procedures have increasingly been performed laparoscopically. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall. However, multiple abdominal incisions are often required in such obesity treatment procedures, thereby increasing chances for undesirable post-operative consequences such as cosmetic scarring.

Gastroplasties have become increasingly favored by surgeons and patients for treating obesity, as well as for treating stomach diseases such as cancer where a portion of the stomach is removed, because gastroplasties do not leave any foreign material in a patient and does not require a complicated intestinal bypass. Instead, the stomach's volume is reduced through partial division of the stomach, thereby leaving a stomach "sleeve" between the esophagus and intestine. A laparoscopic gastroplasty procedure generally involves insufflation of the abdominal cavity with carbon dioxide gas to a pressure of around 15 millimeters of mercury (mm Hg). The abdominal wall is pierced and a 5-10 mm in diameter straight tubular cannula or trocar is then inserted into the abdominal cavity. A laparoscopic telescope connected to an operating room monitor is used to visualize the operative field and is placed through one of the trocar(s). Laparoscopic instruments are placed through two or more additional trocars for manipulation by the surgeon and surgical assistant(s). Thus, such laparoscopic procedures can require multiple instruments to be introduced into a patient through multiple, potentially scarring incisions and/or can result in interference between instruments near each other. The placement of two or more standard cannulas and laparoscopic instruments in the abdomen next to each other and/or placement of two or more instruments into the abdomen through the same incision creates a so-called "chopstick" effect, which describes interference between the surgeon's hands, between the surgeon's hands and the instruments, and between the instruments. This interference greatly reduces the surgeon's ability to perform a described procedure.

Accordingly, there remains a need for methods and devices for performing gastroplasties that minimize patient recovery time, improve cosmetic outcome, and reduce the "chopstick" effect.

### SUMMARY OF THE INVENTION

The present invention generally provides methods and devices for performing gastroplasties. In one embodiment, a surgical method is provided that includes positioning a housing having a plurality of sealing ports in an abdominal wall of a patient, and transecting a greater curvature of a stomach of the patient from a lesser curvature of the stomach using a surgical instrument, e.g., a surgical stapler, inserted through one of the plurality of sealing ports in the housing to form a gastric tube along the lesser curvature that drains into an antrum of the stomach. In some embodiments, the housing can be positioned to form an access hole through an umbilicus of the patient, and the access hole can have a diameter in a range of about 15 to 35 millimeters.

Various instruments can be used to facilitate formation of a gastric tube or stomach sleeve. For example, the method can include visualizing an abdominal cavity of the patient with a scoping device inserted through one of the plurality of sealing ports in the housing. For another example, the method can include transorally introducing a sizing device into the stomach and using the sizing device to size the portion of the stomach to be transected. As another example, the method can include retracting a liver of the patient using a device inserted through one of the plurality of sealing ports in the housing. As yet another example, the method can include tensioning a tissue attached to the stomach using a grasper inserted through one of the plurality of sealing ports in the housing, and detaching at least a portion of the tissue from the stomach using a second surgical instrument inserted through one of the plurality of sealing ports in the housing. The second surgical instrument can include an optically clear distal end to allow visualization of the stomach during detaching of at least a portion of the tissue. As another example, the method can include forming an access hole in the abdominal wall by inserting a trocar through the abdominal wall, and tensioning a tissue attached to the stomach using a grasper inserted through the trocar.

In other embodiments, the method can include bending at least one flexible joint along a longitudinal length of the surgical instrument to position the surgical instrument for transecting. In some embodiments, the method can include, prior to transecting, forming an opening through anterior and posterior walls of the stomach. A location for the opening can be determined by using a scoping device inserted through one of the plurality of sealing ports in the housing to visualize the stomach between the antrum and an angle of His. The method can also include measuring a distance between a pylorus of the patient and the greater curvature of the stomach.

In another embodiment, a surgical method is provided that includes forming a first access hole in an abdominal wall of a patient by positioning a housing having a plurality of sealing ports therein in the abdominal wall, forming a second access hole in the abdominal wall by inserting a trocar through the abdominal wall, and transecting a portion of a stomach of the patient between an opening formed through posterior and anterior walls of the stomach and an angle of His of the stomach using a surgical instrument inserted through one of the housing and the trocar. In some embodiments, the first access hole has a diameter greater than a diameter of the second access hole. The method can have any number of variations. For example, the method can include visualizing an abdominal cavity of the patient with a scoping device inserted through one of the housing and the trocar. As another example, the method can include determining a location for the opening by using a scoping device inserted through one of the housing and the trocar to visualize the stomach between an antrum of the stomach and the angle of His. As still another example, the method can include transorally introducing a sizing device into the stomach and using the sizing device to size the portion of the stomach to be transected. As another example, the method can include bending at least one flexible joint along a longitudinal length of the surgical instrument to position the surgical instrument for transecting. As yet another example, the method can include tensioning a tissue attached to the stomach using a grasper inserted through one of the housing and the trocar, and detaching at least a portion of the tissue from the stomach using a dissecting surgical instrument inserted through one of the housing and the trocar. Tensioning a tissue attached to the stomach can include using at least one additional grasper inserted through the one of the housing and the trocar through which the other grasper is not inserted. The grasper can be inserted through one of the plurality of sealing ports in the housing, and the dissecting surgical instrument can be inserted through another one of the plurality of sealing ports in the housing. The dissecting surgical instrument can include an optically clear distal end to allow visualization of the stomach during detaching of at least a portion of the tissue.

In another embodiment, a surgical method is provided that includes positioning a housing having a plurality of sealing ports in an abdominal wall of a patient to form an access hole through the abdominal wall, transorally inserting a sizing device into a stomach of the patient, using the sizing device to size a portion of the stomach to be transected, and transecting the portion of the stomach using a surgical stapler inserted through the abdominal wall through one of the plurality of sealing ports in the housing. The method can have any number of variations. For example, the method can include inserting a scoping device through the abdominal wall through one of the plurality of sealing ports in the housing and using the scoping device to locate a starting location for the surgical stapler to start transecting the portion of the stomach. For another example, the method can include forming a second access hole in the abdominal wall by inserting a trocar through the abdominal wall, inserting a grasper through the abdominal wall through the trocar, and tensioning a tissue attached to the stomach using the grasper. The method can further include detaching at least a portion of the tissue from the stomach using a dissecting surgical instrument inserted through one of the plurality of sealing ports in the housing.

### BRIEF DESCRIPTION OF THE DRAWINGS

The invention will be more fully understood from the following detailed description taken in conjunction with the accompanying drawings, in which:

FIG. 1 is a perspective partially transparent view of one embodiment of a patient having a multiple port access device positioned in an access hole formed in an abdominal wall of the patient;

FIG. 2 is a perspective view of the multiple port access device of FIG. 1;

FIG. 3 is a partial cross-sectional view of the multiple port access device of FIG. 2;

FIG. 4 is a perspective partially transparent view of the patient of FIG. 1 having a second access hole formed in the umbilicus of the patient;

FIG. 5 is a perspective partially transparent view of the patient of FIG. 4 having a third access hole formed in the abdominal wall of the patient;

FIG. 6 is perspective view of a scoping device inserted into a patient through a multiple port access device and advanced toward a stomach of the patient;

FIG. 7 is a perspective partially transparent view of one embodiment of a liver retracting device retracting a liver of a patient;

FIG. 8 is a perspective partially transparent view of one embodiment of a tacker device shown retracting a liver of a patient;

FIG. 9 is a perspective partially transparent view of one embodiment of a dissecting device dissecting tissue from a stomach of the patient of FIG. 8;

FIG. 10 is a perspective view of one embodiment of a tunnel formed underneath a stomach of a patient;

FIG. 11 is a top view of one embodiment of a dissecting device;

FIG. 12 is a cross-sectional view of a distal hood of the dissecting device of FIG. 11;

FIG. 13 is a cross-sectional view of a shaft of the dissecting device of FIG. 11;

FIG. 14 is a perspective view of one embodiment of a transection starting location on a stomach of a patient;

FIG. 15 is a perspective view of one embodiment of a sealed opening formed at the transection starting location of FIG. 14;

FIG. 16 is a perspective partially transparent view of one embodiment of a transecting device inserted through the multiple port access device of FIG. 1 and about to transect a stomach of the patient;

FIG. 17 is a side view of one embodiment of a transecting device having one flexible joint;

FIG. 18 is a side view of one embodiment of a transecting device having two flexible joints;

FIG. 19 is a side view of one embodiment of a transecting device having two flexible joints and an articulating end effector;

FIG. 20 is a perspective partially transparent view of one embodiment of a sizer advanced into a stomach of a patient;

FIG. 21 is a side cross-sectional view of the patient and sizer of FIG. 20;

FIG. 22 is a perspective view of one embodiment of a transecting device transecting a portion of a stomach of a patient with a sizer positioned in the stomach; and

FIG. 23 is a perspective view of one embodiment of a transected stomach.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for performing gastroplasties. In one embodiment, a method of performing a gastroplasty includes gaining access to a stomach of a patient through an opening formed in the patient's abdominal wall, such as in the umbilicus. A multiple port access device having two or more sealing ports through which surgical instruments can be inserted can be positioned in the abdominal opening. Various instruments can be inserted through the various sealing ports to perform certain steps, such as tensioning and cutting tissue, sizing and transecting the stomach, viewing the surgical site, etc. In certain embodiments, at least one percutaneous opening is formed in the patient's abdominal wall through which one or more surgical instruments can be inserted in addition to any instruments inserted through the multiple port access device. In an exemplary embodiment, the methods and devices are used to perform a Magenstrasse and Mill procedure in which only a portion of the stomach is transected.

A patient can be prepared for a gastroplasty surgical procedure in any way, as will be appreciated by a person skilled in the art. For example, the patient can be fully sedated or consciously sedated for the procedure. Non-limiting embodiments of a conscious sedation system can be found in U.S. Patent Publication No. 2006/0042636 filed on June 21, 2005 and entitled "Oral Nasal Cannula," U.S. Patent No. 6,807,965 issued October 26, 2004 and entitled "Apparatus And Method For Providing A Conscious Patient Relief From Pain And Anxiety Associated With Medical Or Surgical Procedures," U.S. Patent No. 7,201,734 issued April 10, 2007 and entitled "Apparatus For Drug Delivery In Association With Medical Or Surgical Procedures," U.S. Patent No. 7,247,154 issued July 24, 2007 and entitled "Method For Drug Delivery In Association With Medical Or Surgical Procedures," which are hereby incorporated by reference in their entireties.

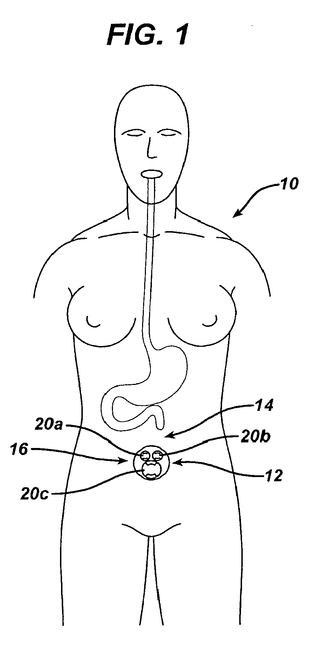

In one exemplary embodiment of a gastroplasty procedure illustrated in FIG. 1, an abdominal opening or access hole 12 is formed in an abdominal wall 14 of a patient 10. FIG. 1 and other figures discussed herein are simplified for ease of presentation and do not always illustrate the patient 10 and/or devices present at a given moment in a surgical procedure, such as devices shown in one or more previously described figures and any additional necessary equipment, e.g., patient monitoring equipment, safety devices, video monitors, etc. Furthermore, the gastroplasty is described as performed by a surgeon, but as will be appreciated by a person skilled in the art, one or more medical professionals, e.g., surgeons, surgical assistants, nurses, etc., can perform any one or more portions of the procedure.

As shown in FIG. 1, the abdominal opening or access hole 12 can be formed in the patient's abdominal wall 14 at an umbilicus of the patient 10, although the abdominal access hole 12 can be formed anywhere in the abdominal wall 14. While a female patient is illustrated, the patient 10 be male or female. Smaller and fewer body cavity incisions can generally improve a patient's recovery time and reduce pain, so it can be advantageous to perform an operation utilizing only a single abdominal incision in the navel. The umbilicus is the thinnest and least vascularized, and a well-hidden, area of the abdominal wall 14. An umbilical incision can be easily enlarged, e.g., in order to eviscerate a larger specimen, without significantly compromising cosmesis and without increasing the chances of wound complications. The abdominal access hole 12 can be in the form of a substantially circular otomy having a diameter in the range of about 15 to 35 mm, but as will be appreciated by a person skilled in the art, the abdominal access hole 12 can have any size and shape. A person skilled in the art will also appreciate that the term "otomy" as used herein is intended to encompass an opening or access hole in a patient that is configured to accommodate an access device with a retractor or other device positionable in the access hole. In certain embodiments, the retractor or other device can have an outer diameter of about 25.4 mm (about 1 inch), although a device need not be inserted through an otomy.

The abdominal access hole 12 can be formed in any way, as will be appreciated by a person skilled in the art. The multiple port access device 16 can be positioned in the abdominal wall 14 following creation of the abdominal access hole 12 in any way such as by using a cutting instrument, e.g., a needle knife, a scalpel, a hook knife, etc. The multiple port access device 16 can have any configuration, but non-limiting embodiments of a multiple port access device can be found in U.S. Patent Publication No. 2006/0247673 filed April 5, 2006 and entitled "Multi-port Laparoscopic Access Device," U.S. Application No. [ ] entitled "Surgical Access Device" [Atty. Docket No. 100873-310 (END6485USNP)] and filed on even date herewith, U.S. Application No. [ ] entitled "Surgical Access Device with Protective Element" [Atty. Docket No. 100873-311 (END6485USNP1)] and filed on even date herewith, U.S. Application No. [ ] entitled "Multiple Port Surgical Access Device" [Atty. Docket No. 100873-312 (END6485USNP2)] and filed on even date herewith, and U.S. Application No. [ ] entitled "Variable Surgical Access Device" [Atty. Docket No. 100873-313 (END6485USNP3)] and filed on even date herewith, which are hereby incorporated by reference in their entireties.

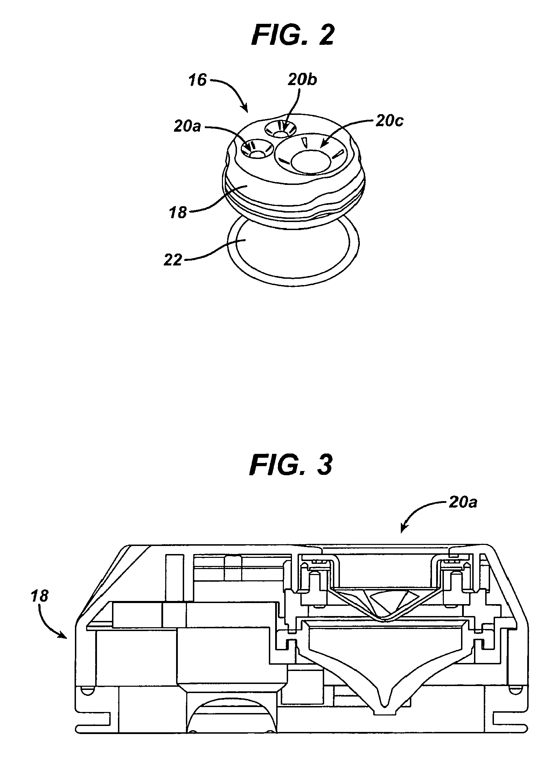

FIGS. 2 and 3 show the multiple port access device 16 of FIG. 1 in more detail. The multiple port access device 16 can have any size, shape, and configuration. For non-limiting example only, the multiple port access device 16 can have a size configured to allow the multiple port access device 16 to be positioned in an abdominal incision having a diameter in the range of about 15 to 35 mm. The illustrated multiple port access device 16 includes a housing 18 having three ports 20a, 20b, 20c extending therethrough, although the multiple port access device 16 can have any number of ports. In addition, the ports 20a, 20b, 20c can have the same size or varying sizes configured to provide for the insertion of differently sized surgical instruments therethrough. In an exemplary embodiment, the ports 20a, 20b, 20c can each be sized to provide for instruments preferably less than or equal to about 5 mm in diameter by having diameters of about 5 mm, preferably with at least one of the ports, e.g., the smaller ports 20a, 20b as illustrated, having a diameter of about 3 mm. The ports 20a, 20b, 20c can have various angular orientations and can each include one or more sealing elements configured to provide a seal to prevent the escape of insufflation gas and/or to form a seal around an instrument inserted therethrough. Any seal(s) can be used, as will be appreciated by a person skilled in the art, such as duck bill valves, iris seals, zero-closure valves, gaskets, gel seals, cone seals, diaphragm seals, etc. The multiple port access device 16 also includes a retractor 22 coupled to and extending distally from the housing 18. The retractor 22 is configured to be positioned within an opening in tissue to form a pathway through the tissue for surgical instruments inserted through the ports 20a, 20b, 20c. While the retractor 22 can have a variety of configurations, in the illustrated embodiment the retractor 22 has an elongate cylindrical shape with proximal and distal flanges that can engage tissue therebetween. The retractor 22 also has a lumen extending therethrough and in communication with each of the ports 20a, 20b, 20c.

In general, the multiple port access device used in the gastroplasty can provide access to a patient's abdominal cavity by positioning the multiple port access device within an access hole or opening in the patient's body. For non-limiting example, in the illustrated embodiment of FIGS. 2 and 3, the retractor 22 of the multiple port access device 16 can be positioned in the patient's body such that the distal flange of the retractor 22 is positioned adjacent to an internal surface of the patient's abdominal wall and the proximal flange of the retractor 22 is positioned adjacent to the patient's skin on an exterior of the patient's body. The retractor's elongate portion can form a pathway through the opening in the patient's body so that surgical instruments can be inserted from outside the body into the abdominal cavity. The elasticity of the skin of the patient can assist in the retention of the retractor 22 in the opening made in the body. In one embodiment, the retractor 22 can be substantially flexible so that it can easily be maneuvered into and within tissue as needed.

Optionally, one or more openings or access holes in addition to the abdominal access hole 12 can be formed in the patient's abdominal wall 14. Each additional abdominal access hole can have any size, shape, and configuration, but in an exemplary embodiment, the additional abdominal access hole(s) are each percutaneous openings. A person skilled in the art will appreciate that the term "percutaneous opening" or "percutaneous access hole" as used herein is intended to encompass a relatively small opening or access hole in a patient that preferably has a diameter in a range of about 3 to 5 mm. Any of the additional abdominal access hole(s) can be formed before and/or after the abdominal access hole 12, but in an exemplary embodiment, any additional abdominal access hole(s) are formed after the abdominal access hole 12 to allow prior insufflation of the patient's abdominal cavity using a surgical device inserted through the abdominal access hole 12, as discussed further below.

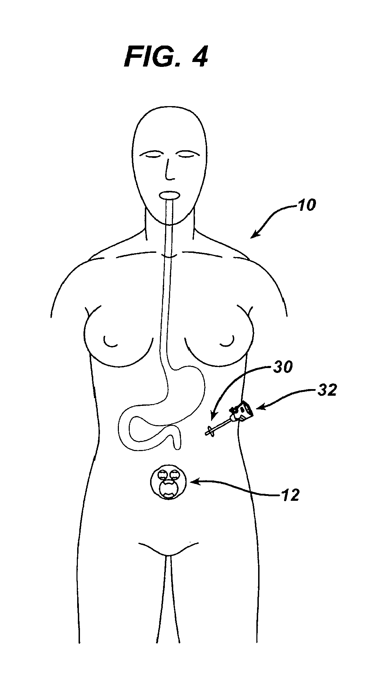

FIG. 4 illustrates one embodiment of an additional abdominal opening or access hole 30 formed in the patient 10 in addition to the abdominal access hole 12 having the multiple port access device 16 positioned therein. The additional abdominal opening 30 can have any size, shape, and configuration, but in an exemplary embodiment, the additional abdominal access hole 30 is a percutaneous opening having a diameter of about 5 mm and configured to allow passage of a surgical instrument, e.g., a trocar, a scoping device, a surgical stapler, a clip applier, etc., therethrough. As will be appreciated by a person skilled in the art, the additional abdominal access hole 30 can be formed in any way through the patient's abdominal wall 14 to provide access to the patient's abdominal cavity. In an exemplary embodiment, the surgeon can insert a trocar 32 through the abdominal wall 14 to form the additional abdominal access hole 30. As will be appreciated by a person skilled in the art, the surgeon can use any size and type of trocar to form the additional abdominal access hole 30. The trocar 32 can be configured to allow a rigid or flexible surgical instrument, e.g., a grasper, a cutting instrument, a scoping device, etc., to be passed therethrough and into the patient's abdominal cavity. A person skilled in the art will appreciate that the term "grasper" as used herein is intended to encompass any surgical instrument that is configured to grab and/or attach to tissue and thereby manipulate the tissue, e.g., forceps, retractors, movable jaws, magnets, adhesives, stay sutures, etc.

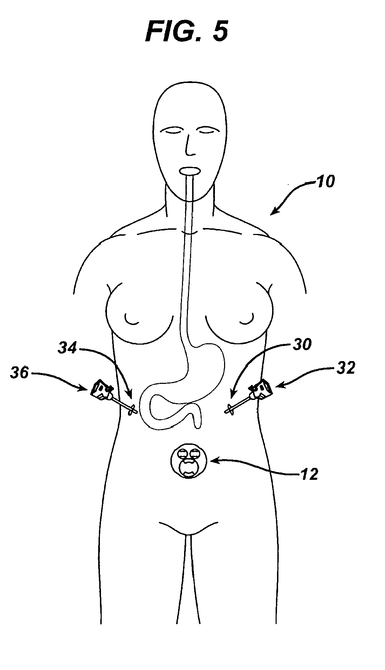

FIG. 5 shows another embodiment where, in addition to the abdominal access hole 12 and the additional abdominal access hole 30, a second additional abdominal opening or access hole 34 is formed in the patient's abdominal wall 14 to provide access to the patient's abdominal cavity. The second additional abdominal opening 34 can have any size, shape, and configuration, but in an exemplary embodiment, the second additional access hole 34 has a size, shape, and configuration substantially the same as the abdominal access hole 30. The additional abdominal access holes 30, 34 can be formed in any order with respect to one another and with respect to the abdominal opening 12 with the multiple port access device 16 positioned therein. The additional abdominal access holes 30, 34 can be positioned anywhere through the patient's abdominal wall 14, but as illustrated, the additional abdominal access holes 30, 34 can be substantially laterally aligned on opposed sides of the patient's abdomen. The access hole 12 having the multiple port access device 16 positioned therein can, as illustrated, be non-laterally aligned with and be located between the additional abdominal access holes 30, 34, e.g., in the umbilicus. In this way, a grasper can be inserted through at least one of the additional abdominal access holes 30, 34 and can allow tissue to be tensioned in the patient 10 at a transverse angle relative to a surgical instrument, e.g., a cutting instrument, inserted into to the patient 10 through the umbilicus. As will be appreciated by a person skilled in the art, the second additional access hole 34 can be formed in any way through the patient's abdominal wall 14 to provide access to the patient's abdominal cavity, but in an exemplary embodiment it is formed using a trocar 36 in a way similar to that discussed above for the other additional abdominal opening 30 created using the trocar 32. The trocars 32, 36 inserted through the additional abdominal openings 30, 34 can include any trocar, same or different from each other.

As will be appreciated by a person skilled in the art, access holes through the abdominal wall can be formed in any way. Non-limiting embodiments of a trocar that can be used to form an abdominal access hole can be found in U.S. Patent Publication No. 2007/0260273 filed on May 8, 2006 and entitled "Endoscopic Translumenal Surgical Systems," which is hereby incorporated by reference in its entirety. An exemplary embodiment of a trocar can include a trocar housing configured to allow a surgical device to pass therethrough, and a trocar sleeve or overtube mated to or extending from the trocar housing. The trocar can also include an obturator configured to pass through the trocar housing and the trocar sleeve. The obturator can have an inner lumen formed therethrough for receiving a scoping device and/or other surgical device therein, and a distal end configured penetrate through tissue. The trocar sleeve can be slidably disposed over the obturator and can function as a placeholder after the trocar is inserted through tissue and the obturator is removed. Non-limiting embodiments of a sleeve and an obturator that can be used to form an abdominal access hole can be found in U.S. Patent Application No. [ ] filed on even date herewith and entitled "Methods And Devices For Performing Gastrectomies And Gastroplasties," [Atty. Docket No. 100873-317 (END6488USNP)], which is hereby incorporated by reference in its entirety.

Once access to the abdominal cavity is obtained, the surgeon can insufflate the patient's abdominal cavity through an opening in the patient's abdomen, as will be appreciated by a person skilled in the art, to expand the abdominal cavity and provide a larger, more easily navigable surgical workspace. In an exemplary embodiment, the surgeon can insufflate the abdominal cavity by passing a fluid under pressure, e.g., nontoxic carbon dioxide gas, through at least one of the sealing ports 20a, 20b, 20c in the multiple port access device 16. The fluid can have a pressure in the range of about 10 to 15 mm Hg, or any other pressure, as will be appreciated by a person skilled in the art. As mentioned above, the multiple port access device 16 can include one or more sealing elements that prevent the insufflation fluid from escaping the abdominal cavity through the multiple port access device 16. If one or more openings in addition to the abdominal access hole 12 having the multiple port access device 16 positioned therein are formed through the patient's abdominal wall 14 and have a surgical device, e.g., a trocar, extending therethrough, the device can be configured to provide a seal that prevents the insufflation fluid from escaping the abdominal cavity therethrough. A non-limiting example of a sealing trocar that does not use seals is the SurgiQuest AirSeal^{™} available from SurgiQuest, Inc. of Orange, Connecticut.

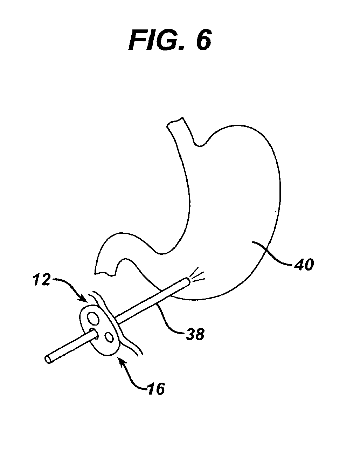

Any number of scoping devices can be advanced through any one or more openings or access holes (natural or surgically created) into the patient 10 to provide visualization inside the patient's body during the surgical procedure. In an exemplary embodiment, as shown in FIG. 6, a scoping device, e.g., a laparoscope 38, can be inserted into one of ports 20a, 20b, 20c in the multiple port access device 16 positioned in the abdominal access hole 12, and it can be advanced toward a stomach 40 of the patient 10. A person skilled in the art will also appreciate that a scoping device used in the gastroplasty can include any surgical device having a viewing element, e.g., a lens, located thereon. Non-limiting examples of a scoping device include an endoscope, a laparoscope, a gastroscope, and a colonoscope. The laparoscope 38 can be inserted into the multiple port access device 16 at any time, including during penetration through the tissue or after the multiple port access device 16 penetrates the abdominal wall 14, and the laparoscope 38 can be inserted through one of the ports 20a, 20b, 20c after insufflation of the patient's abdominal cavity. The laparoscope 38 and/or another scoping device can be inserted and removed from the patient 10 through the multiple port access device 16 and/or other body openings at any one or more times during the surgical procedure The laparoscope 38, as well as the other devices discussed herein, can be made from any combination of rigid and/or flexible materials, but in an exemplary embodiment the materials are biocompatible. A person skilled in the art will appreciate that the term "flexible" as used herein is intended to encompass a variety of configurations. Generally, a "flexible" member has some degree of elasticity, e.g., is capable of bending without breaking. In an exemplary embodiment, a flexible device or at least portions thereof are composed of at least one biocompatible and flexible material, e.g., plastic, titanium, stainless steel, etc. Various portions of a flexible device can also be formed from a shape memory material, such as Nitinol. The laparoscope 38 can work in cooperation with device(s) advanced into the patient 10, e.g., through any one or more of the other ports 20a, 20b, 20c in the multiple port access device 16, to provide visualization of the device(s), where the flexibility of the laparoscope 38 can improve the angle of visualization of the device(s) and allow the laparoscope 38 to flex away from the operative site to help reduce interference of the laparoscope 38 with operation of the device(s) in the body.

In one embodiment, a scoping device inserted into the patient 10 can include one or more distal, flexible joints that can help orient the scoping device inside the patient 10. Non-limiting embodiments of flexible joints on a surgical device can be found in previously mentioned U.S. Patent Application No. [ ] filed on even date herewith and entitled "Methods And Devices For Performing Gastrectomies And Gastroplasties," [Atty. Docket No. 100873-317 (END6488USNP)]. In general, the flexible joint(s) can be configured to flex or bend. The flexible joint(s) can be passively actuated, e.g., moveable when abutted by one or more adjacent structures, and/or actively actuated, e.g., through manipulation of a mechanical and/or manual actuation mechanism. The flexible joint(s) can be configured to bend in a single direction when actuated, and the single direction can be selectively chosen, e.g., left, right, up, down, etc. If a surgical device includes a plurality of flexible joints, each of the flexible joints can be configured to be independently actuated in any direction same or different from any of the other flexible joints of the surgical device. The actuation mechanism can be configured to control the amount of movement in a chosen direction. The flexible joint(s) can be formed in any way, same or different from one another, as will be appreciated by a person skilled in the art. For non-limiting example, the flexible joint(s) can be made from a flexible material, can include one or more features formed therein to facilitate flexibility, e.g., a plurality of cut-outs, slots, etc., and/or can be formed from a plurality of linkages that are movably coupled to one another. In an alternate embodiment, a scoping device can have two or more flexible joints each at different locations along its longitudinal axis, with or without use of a sleeve, to allow the scoping device to bend in at least two directions relative to the scoping device's longitudinal axis. A non-limiting example of a multibending scoping device is the R-Scope XGIF-2TQ260ZMY available from Olympus Corp. of Tokyo, Japan.

During the surgical procedure, the patient's stomach can be difficult to adequately access. The patient's liver can be retracted during the gastroplasty to help the surgeon gain better access to the patient's stomach. Although the liver can be retracted at any time during the surgical procedure, in an exemplary embodiment the liver is retracted after insertion into the patient 10 of a scoping device, e.g., the laparoscope 38 through the multiple port access device 16, to provide visualization of the abdominal cavity before and during retraction of the liver. The liver can be retracted in any way appreciated by a person skilled in the art, but the liver is preferably retracted using at least one device inserted into the abdominal cavity of the patient 10 through, e.g., the previously-formed abdominal access hole 12, through another abdominal opening, etc. Also as will be appreciated by a person skilled in the art, a draining device, e.g., a penrose drain, a Jackson-Pratt drain, etc., can be disposed in the patient's abdominal cavity to help hold the liver and/or drain excess fluid that can accumulate in the abdominal cavity during the surgical procedure, particularly following liver retraction.

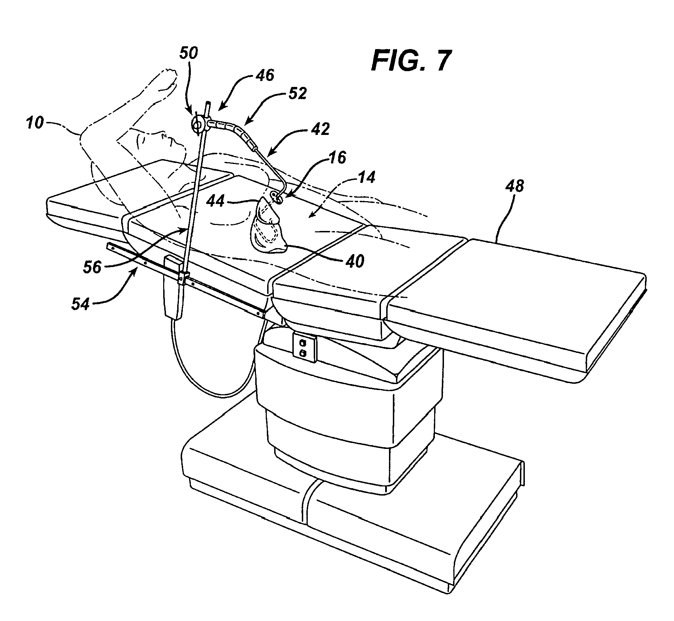

In an exemplary embodiment, a retractor device, such as a Nathanson liver retractor, can be used to retract the patient's liver. FIG. 7 illustrates one embodiment of a liver retraction procedure using a Nathanson liver retractor 42 to retract a liver 44 of the patient 10 away from the stomach 40 of the patient 10. As will be appreciated by a person skilled in the art, the surgeon can use the Nathanson liver retractor 42 to "hook" the liver 44 and hold the liver 44 away from the stomach 40 in a desired retracted position. The Nathanson liver retractor 42 can be inserted through one of the ports 20a, 20b, 20c, e.g., one of the smaller ports 20a, 20b, in the multiple port access device 16 in the abdominal access hole 12 as illustrated, or the Nathanson liver retractor 42 can be advanced into the patient 10 in another way, such as through a cannulated device providing access into the patient's abdominal cavity, e.g., through the trocar 30 of FIG. 4. Although not shown in FIG. 7, the patient's abdominal cavity can be visualized during liver retraction using a scoping device, e.g., the laparoscope 38, advanced through one of the multiple port access device's ports 20a, 20b, 20c, e.g., the larger of the ports 20c. A grasper (not shown) can be advanced through the abdominal wall 14, e.g., directly, through the multiple port access device 16, through a trocar, via a working channel of a scoping device, etc., to assist in retracting the liver 44 and/or otherwise assist in the gastroplasty.

Optionally, as illustrated in FIG. 7, a support 46 external to the patient 10 can be used to mount the Nathanson liver retractor 42 to an examination table 48 on which the patient 10 rests, although any other support can be used if a support is used at all for a liver retractor. By mounting the Nathanson liver retractor 42, the surgeon does not need to continuously hold the Nathanson liver retractor 42 in place during the surgical procedure, thereby freeing the surgeon to attend to other surgical matters, and/or reducing the required number of operating room personnel. Non-limiting embodiments of a support can be found in previously mentioned U.S. Patent Application No. [ ] filed on even date herewith and entitled "Methods And Devices For Performing Gastrectomies And Gastroplasties," [Atty. Docket No. 100873-317 (END6488USNP)]. The support can have a variety of sizes, shapes, and configurations, but as illustrated, the support 46 can include an adapter 50 and a flexible arm 52 configured to couple to the mounted device and configured to be coupled at a terminal end thereof to the adapter 50. The flexible arm 52 is generally configured to be movable, as will be appreciated by a person skilled in the art, to allow the mounted device's position to be adjusted relative to the examination table 18. The adapter 50 can be movable and can mate, as shown, to a table mount coupled to the examination table 48 and including a table rail 54 and a bracket 56 coupled at its respective terminal ends to the table rail 54 and the adapter 50. In an alternate embodiment, in addition to or instead of the examination table 18, the support can mount to another stable structure near the patient 10, e.g., a wall, the ceiling, an independent structure standing on the floor similar to an IV pole or a microphone stand, an overhead fixture, etc. The Nathanson liver retractor 42 can be mounted at any time during the gastroplasty procedure, and its mounting can be re-adjusted and/or released at any time, but in an exemplary embodiment, the Nathanson liver retractor 42 is mounted before arranging the liver 44 into a desired retracted location in the patient 10. The Nathanson liver retractor 42 and/or the support 46, e.g., the flexible arm 52, the adapter 50, and/or the bracket 56, can be adjusted to help move the liver 44 to its desired retracted location.

A person skilled in the art will appreciate that a support can be used to mount the Nathanson liver retractor 42 and/or any other surgical instrument used during the gastroplasty that does not require constant hands-on manipulation. Multiple supports can be used in a single surgical procedure.

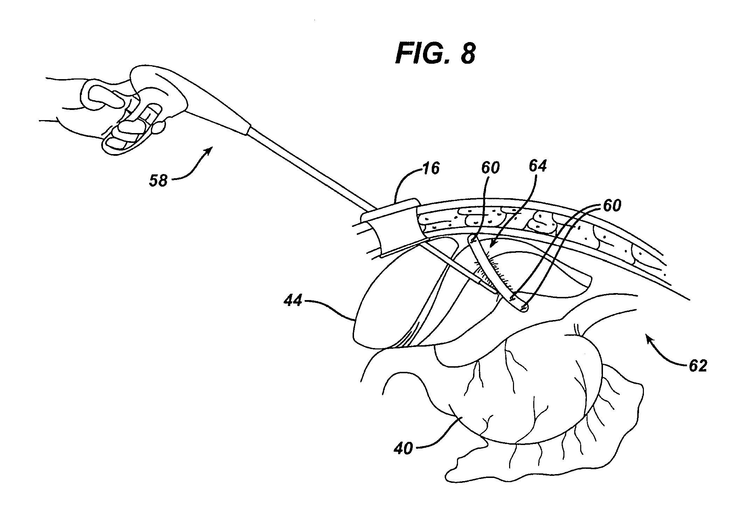

FIG. 8 illustrates an alternate embodiment of a liver retraction procedure that uses a tacker device 58 inserted through the multiple port access device 16 to help retract the patient's liver 44 to a desired location away from the patient's stomach 40. As will be appreciated by a person skilled in the art, the tacker device 58 can deliver and apply one or more tacks 60 and/or mesh to an abdominal cavity 62 of the patient 10 to lift and support the liver 44. FIG. 8 shows tacks 60 applying a penrose drain 64 to help drain fluid away from the surgical site, which in this embodiment can also serve as a retractor device to help hold the liver 44 in its desired retracted position.

In another embodiment, the surgeon can introduce into the patient 10 suture anchors, e.g., t-tags, hooks, etc., having sutures attached thereto. The sutures can be attached to the liver 44, tensioned to desirably position the liver 44, and extracorporeally tied or otherwise secured to maintain the liver 44 in a desired position. In still another embodiment, the liver 44 can be retracted using magnets. The surgeon can affix one or more internal magnets to the liver 44 and one or more external magnets on an outside surface of the patient's abdomen wall 14. The external magnets can attract the internal magnets, thereby moving the liver 44 toward an inner surface of the abdominal wall 14. A liver retracting device can be used alone or in combination with any one or more other liver retracting devices, e.g., magnets in combination with tackers and mesh, a Nathanson liver retractor in combination with suture anchors and sutures, a Nathanson liver retractor in combination with a surgical adhesive, etc.

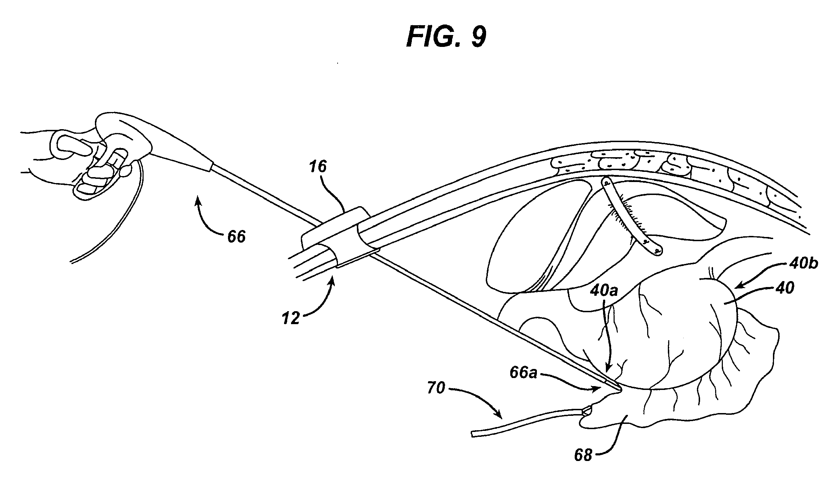

Prior to transecting the stomach 40, the stomach 40 can be separated from tissue attached to the stomach 40, e.g., an omentum, vessels, any adhesions on the stomach 40, etc., to free a fundus of the stomach 40. As will be appreciated by a person skilled in the art, the tissue attached to the stomach 40 can be separated from the stomach 40 using any one or more dissecting devices. A person skilled in the art will also appreciate that the term "dissector," "dissecting device," or "dissecting surgical instrument" as used herein is intended to encompass any surgical instrument that is configured to cut tissue, e.g., a scalpel, a harmonic scalpel, a blunt dissector, a cautery tool configured to cut tissue, scissors, an endoscopic linear cutter, a surgical stapler, etc. The desired tissue can be separated from the stomach 40 in any way, but in an exemplary embodiment the surgeon cuts adjacent to the greater curvature of the stomach 40 to free the fundus from the omentum. The dissector can be introduced into the patient 40 through any access hole (natural or surgically created). In one embodiment shown in FIG. 9, a dissector 66 can be inserted through the multiple port access device 16 in the abdominal access hole 12 and used to cut an omentum 68 from the stomach 40. As shown in this illustrated embodiment, the dissector 66 has an end effector 66a with a distal end having a pair of movable jaws configured to cut tissue. With the desired tissue dissected, a posterior of the stomach 40 can be visualized and/or accessed between an antrum 40a of the stomach 40 and an angle of His 40b of the stomach 40.

In an exemplary embodiment, the omentum 68 and/or any other desired tissue can be tensioned using a grasper 70 while the dissector 66 dissects tissue from the stomach 40. The grasper 70 can be introduced into the patient 10 in any way, such as through the multiple port access device 16, but in an exemplary embodiment, the grasper 70 can be inserted through a percutaneous abdominal opening, e.g., through the trocar 32 of FIG. 4. Generally, the surgeon can pass tissue from the dissector 66 to the grasper 70, grasp the tissue with the grasper 70, pull the grasper 70 to tension the grasped tissue, and dissect tissue using the dissector 66. The surgeon can repeat this process any number of times to free the stomach fundus. Although only one grasper is shown in the embodiment illustrated in FIG. 9, the surgeon can use any number of graspers, which can be inserted in any way into the patient's abdominal cavity. If a scoping device, e.g., the laparoscope 38, is inserted into the patient's abdominal cavity, the surgeon can use the scoping device to provide visualization to, e.g., help position the grasper 70 and/or an additional grasper. Alternatively or in addition, a scoping device can visualize the posterior of the stomach 40 during and/or after dissection of desired tissue.

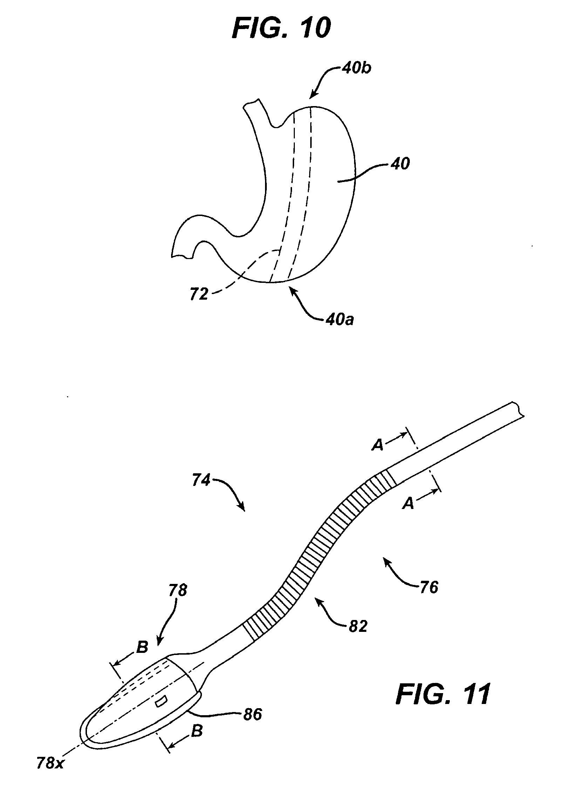

In an alternate exemplary embodiment illustrated in FIG. 10, a dissector can be used to form an opening 72 under the stomach 40. The opening 72 can have any size, shape, and configuration, but in the illustrated exemplary embodiment, the opening 72 can include a tunnel having a substantially constant diameter along its longitudinal length and having a substantially circular cross-sectional shape. The surgeon can visualize the posterior of the stomach 40 from the antrum 40a to the angle of His 40b by, e.g., advancing a scoping device through at least a partial longitudinal length of the opening 72.

FIGS. 11-13 illustrate one embodiment of a dissector 74 that can be used to form the opening 72, although as will be appreciated by a person skilled in the art, any dissector can be used to create the opening 72. The dissector 74 can be rigid, flexible, or any combination thereof. The dissector 74 can include an elongate shaft 76 having a hood 78 at a distal end thereof configured to penetrate tissue. The hood 78 can be integrally formed with the shaft 76, or the hood 78 can be configured as an end cap attached to the shaft 76. The dissector 74 can also include a handle (not shown) at its proximal end configured to allow manipulation and/or actuation of the dissector 74.

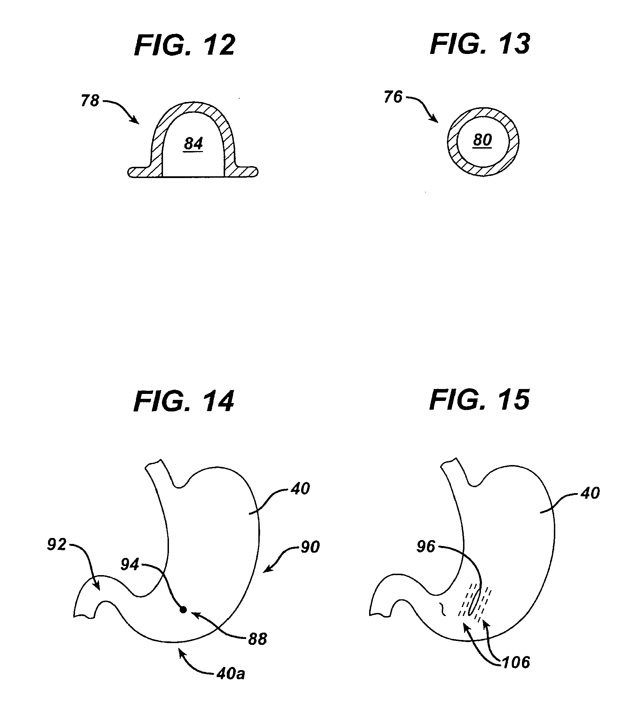

The shaft 76 can have any size, shape, and configuration, but as illustrated in this exemplary embodiment, the shaft 76 can have a substantially cylindrical shape and have a substantially circular cross-section A-A as best seen in FIGS. 11 and 13. The shaft 76 can be solid, hollow, or any combination thereof. In the illustrated embodiment, the shaft 76 has an inner lumen 80 extending therethrough through which a surgical instrument can be passed. The shaft 76 can also include a seal (not shown) to provide a fluid seal around any instrument passed through the inner lumen 80. In an exemplary embodiment, the shaft 76 has at least one flexible joint 82 configured to allow that portion of the shaft 76 to flex or bend. The flexible joint 82 can be configured in any way, as discussed above. The flexible joint 82 can extend along any portion of the shaft's longitudinal length and can be passively and/or actively actuated, e.g., by using an actuation control mechanism located at the dissector's handle.

The dissector's hood 78 can also have any size, shape, and configuration. In an exemplary embodiment, the hood 78 is rigid and distally tapered with a rounded, bullet shaped tip, as best seen in FIG. 11 and in a hood cross-section B-B in FIG. 12, to help the hood 78 more easily penetrate tissue. The hood 78 preferably has a width larger than a width of the shaft 76 to more easily allow the shaft 76 to pass through tissue penetrated by the hood 78. The hood 78 can be opaque, semi-transparent, and/or optically clear, but in an exemplary embodiment, at least a portion of the hood 78 is made from an optically clear material. The hood 78 can have a hollow interior 84 that can be in communication with the shaft's inner lumen 80 to allow a surgical instrument to extend through the inner lumen 80 and into the hood's hollow interior 84. If the hood 78 is at least partially optically clear, a scoping device can be advanced through the shaft's inner lumen 80 and into the hood's hollow interior 84 where it can provide visualization of a surgical site through the hood 78. The hood 78 can optionally include an opening extending through a surface thereof that is in communication with the hollow interior 84, which can allow a surgical instrument such as a scoping device, a cutting instrument, or a dissecting instrument to be advanced therethrough to cut tissue and/or provide visualization.

The hood 78 can be substantially smooth, or as illustrated, the hood 78 can include a penetrating element 86, e.g., a sharp knife edge, a beveled edge (including a chamfered edge), a pointed needle, an electronic cutter, a paddle, etc., that can protrude outward from an outer surface thereof to help the hood 78 penetrate tissue. The penetrating element 86 can be located anywhere on the hood 78, but as shown, the penetrating element 86 extends around a perimeter of the hood 78. The penetrating element 86 as shown is in the form of a paddle that does not necessarily cut tissue but rather merely extends outward from an outer surface of the hood 78. The paddle can have a generally planer, elongate configuration, and in use it can be configured to penetrate tissue with or without the tissue having a previously formed cut or slit therein, e.g., formed with a cutting instrument inserted through the dissector 74. For example, the paddle can be rotated to spread open an elongate cut made through tissue. A person skilled in the art will appreciate that the penetrating element 86 can be formed integrally with the hood 78 such that the hood 78 and the penetrating element 86 are formed as a single piece of material, or it can be separate from and mated to the hood 78.

In use, the dissector 76 can be introduced to a surgical site in any way appreciated by a person skilled in the art, e.g., advanced through a trocar or access device, advanced via a working channel of a scoping device, etc. Advancing the hood 78 through tissue desired for dissection can dissect the tissue by, e.g., separating tissue layers and/or creating an incision, thereby allowing the hood 78 and/or at least a portion of the shaft 76 to be advanced through the tissue. The hood 78 can be manually and/or mechanically rotatable around a central axial axis 78x of the hood 78 to help the hood 78 penetrate tissue by, e.g., rotating a proximal handle of the dissector 74, as will be appreciated by a person skilled in the art.

As mentioned above, the surgeon can use a surgical instrument such as a scoping device to visualize the posterior of the stomach 40. Such visualization can help in determining a starting location 88 for a transection of the stomach 40, as illustrated in FIG. 14. The starting location 88 can be located anywhere on the stomach 40 and can be determined in any way, as will be appreciated by a person skilled in the art. For example, a distance can be measured along a greater curvature 90 of the stomach 40 from a pylorus 92 of the stomach 40, and in an exemplary embodiment from a pyloric sphincter or valve of the pylorus 92, to determine the starting location 88. In an exemplary embodiment, the starting location 88 has a lateral distance from the pylorus 92 in a range of about 2 to 6 centimeters (cm) and has an axial distance from the antrum 40a of about 2 cm. The surgeon can mark the starting location 88 in any way, such as by mentally marking or remembering the starting location or by applying a marker. As will be appreciated by a person skilled in the art, any marker can be used to mark the starting location, e.g., a mark using electrocautery, a mark using a harmonic scalpel, an ink marker 94 applied in any way appreciated by a person skilled in the art, such as via a marking device inserted through an abdominal or other access hole, etc.

Transection of the stomach 40 can begin substantially at the starting location 88. In an exemplary embodiment illustrated in FIG. 15, an opening 96 can be created through anterior and posterior walls of the stomach 40 substantially at the starting location 88. The opening 96 can more easily allow a transection device to be desirably positioned with respect to the stomach 40 when the transection device begins transecting the stomach 40 between the antrum 40a and the angle of His 40b. The opening 96 can have any size and shape, e.g., substantially circular, etc.. The opening 96 can be closed or sealed to help prevent bleeding and/or prevent fluid or debris seepage between the stomach 40 and the patient's abdominal cavity. Having a closed opening can also provide the surgeon with increased flexibility during the surgical procedure because the surgeon can create the opening 96 without immediately transecting the stomach 40 thereafter but instead first, e.g., size the stomach 40 as discussed further below. The opening 96 can be closed in any way, as will be appreciated by a person skilled in the art, such as by applying one or more securing elements, e.g., staples 106 as shown, sutures, glues such fibron glues, pledgets, etc. The securing element(s) can be applied following creation of the opening 96 and/or the device that creates the opening 96 can also be configured to apply one or more securing elements when it forms the opening 96.

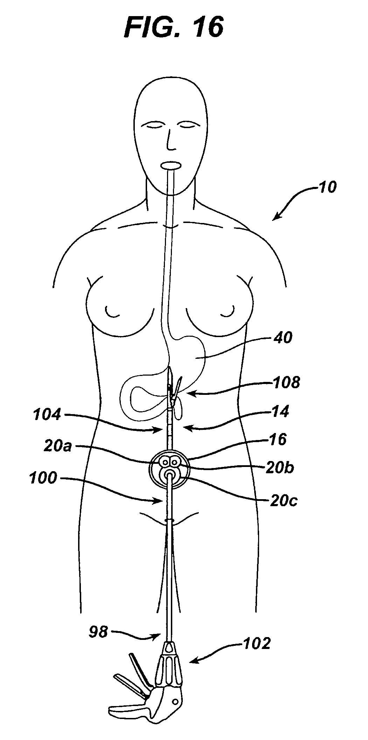

As will be appreciated by a person skilled in the art, the opening 96 can be formed in any way using any surgical device, e.g., a cutting instrument, a dissector, a transector, etc.. In an exemplary embodiment illustrated in FIG. 16, the surgeon can advance a transector into the patient's abdominal cavity through one of the sealing ports 20a, 20b, 20c, e.g., the larger port 20c, of the multiple port access device 16 positioned in the patient's abdominal wall 14. As will be appreciated by a person skilled in the art, the opening 96 can be formed and the stomach 40 can be transected using any one or more transecting devices. A person skilled in the art will also appreciate that the term "transector," "transecting device," or "transecting surgical instrument" as used herein is intended to encompass surgical devices that alone or in combination can cut and secure tissue, e.g., a surgical stapler configured to cut and staple tissue. In an exemplary embodiment, a first transector, such as a circular surgical stapler, is used to apply one or more circular rows of staples and to cut the stapled tissue leaving an opening formed through the tissue that is surrounded by staples. Non-limiting embodiments of a circular surgical stapler can be found in U.S. Patent No. 5,285,945 issued February 14, 1995 and entitled "Surgical Anastomosis Stapling Instrument," which is hereby incorporated by reference in its entirety. As will be discussed in more detail below, a second transector 98, such as a linear surgical stapler as shown, can be introduced into the patient 10 through any opening, e.g., through an abdominal access hole, a natural orifice, etc., with or without a single or multiple port access device positioned therein. The second transector 98 can be passed through the opening 96 in the stomach to position tissue to be transected between opposed jaws of the transector. In the illustrated embodiment, the transector 98 includes a handle assembly 102 at its proximal end having an elongate shaft 100 extending therefrom and configured to be at least partially inserted into a patient's body. An end effector 108 at a distal end of the shaft 100 is configured to cut tissue and to apply one or more rows of staples to secure the cut tissue. As will be appreciated by a person skilled in the art, at least one handle included in the handle assembly 102 can be movable to cut tissue and/or apply one or more staples.

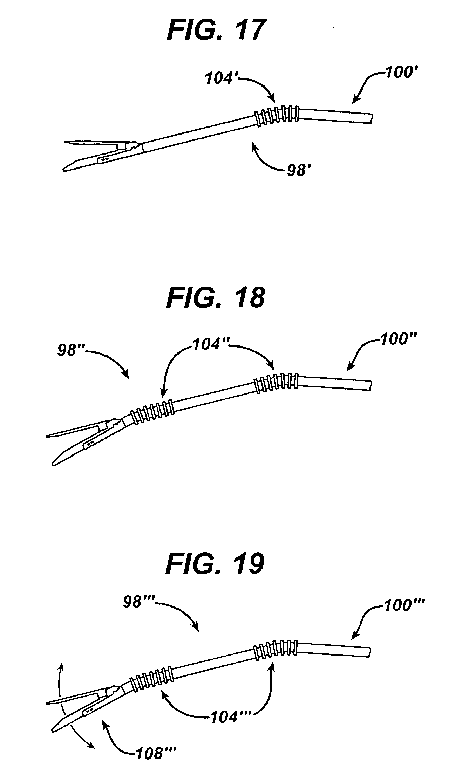

The transector(s) can be rigid, flexible, or a combination thereof. In an exemplary embodiment, referring to transector 98 by way of non-limiting example, the transector 98 includes at least one flexible joint 104 along its elongate shaft 100, preferably in a distal portion thereof as illustrated that articulates to allow pitch and/or yaw displacement of the shaft 100 distal to the flexible joint 104. The flexible joint 104 can be articulated in any way, passively and/or actively, such as by actuating a control mechanism at the transector's handle 102. The flexible joint 104 can be configured in any way, as discussed above. One embodiment of a transector 98' including a single flexible joint 104' at a distal end of the transector's elongate shaft 100' is illustrated in FIG. 17, while another embodiment of a transector 98" including two flexible joints 104" at a distal end of the transector's elongate shaft 100" is illustrated in FIG. 18. FIG. 19 illustrates another embodiment of a transector 98''' having two flexible joints 104''' at a distal end of its elongate shaft 100''' and having an end effector 108''' configured to be movable to allow pitch and/or yaw displacement of the end effector 108''' by having a flexible joint substantially at a coupling of its shaft 100''' and end effector 108'''. A person skilled in the art will appreciate that flexible joints can also pivot or more about a single point, rather than flexing along a length of the shaft.

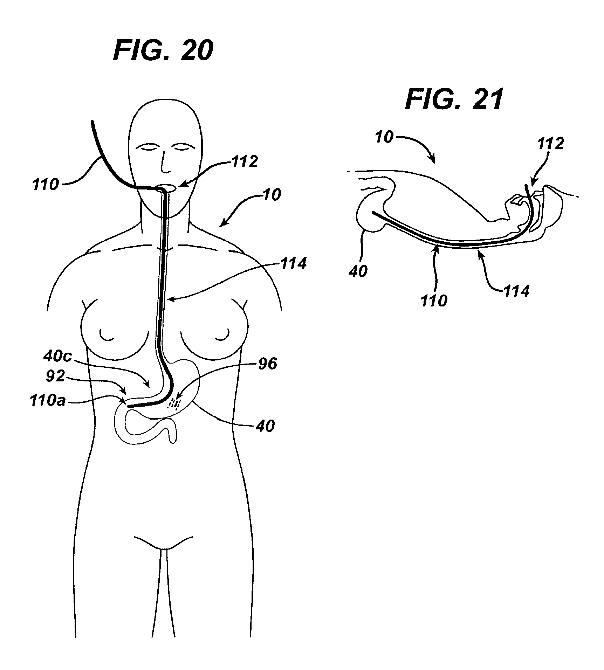

At any time prior to transecting the stomach 40, the surgeon can manipulate the stomach 40 to form a gastric tube or stomach sleeve in the stomach 40. In an exemplary embodiment, the stomach sleeve can be formed after creation of the tunnel 72 under the stomach 40 and the opening 96 through the stomach 40, although the sleeve can be formed before or after creation of the tunnel 72 or the opening 96. As illustrated in FIGS. 20 and 21, the surgeon can introduce a sizing device 110 into the stomach 40 to help size the portion of the stomach 40 that will form the stomach sleeve. The sizing device 110 can be introduced into the stomach 10 in any way, but in this illustrated exemplary embodiment, the sizing device 110 is transorally introduced into the stomach 40, e.g., through a mouth 112 and an esophagus 114 of the patient 10. A person skilled in the art will appreciate that the term "sizer," "sizing device," or "sizing instrument" as used herein is intended to encompass any surgical instrument, e.g., a bougie, a scoping device, a catheter, etc, that is configured to indicate a desired gastric sleeve area. The sizer 110 can optionally include a light at its distal end to help the surgeon advance the sizer 110 through the esophagus 114 and desirably position the sizer 110 in the stomach 40. The sizer's size and shape can generally correspond to a size and shape of the stomach sleeve desired to be formed in the patient 10, so the surgeon can choose a sizer having any size, shape, and configuration that generally corresponds to the desired sleeve dimensions. In an exemplary embodiment, the sizer 110 is a flexible surgical instrument having a substantially cylindrical shape and a substantially constant diameter along the sizer's longitudinal length in the range of about 28 to 42 French (about 9.3 to 14 mm).

The sizer 110 can be adjusted in the stomach 40 to place the sizer 110 in a sizing position that generally indicates the size and position of the stomach sleeve following at least partial transection of the stomach 40. In an exemplary embodiment, the sizer 110 in the sizing position extends along a lesser curvature 40c of the stomach 40 and into the pylorus 92 of the stomach 40 so at least a distal-most end 11a of the sizer 110 extends to the pyloric sphincter or valve of the pylorus 92. The sizer 110 can be adjusted in the patient 10 in any way, as will be appreciated by a person skilled in the art. In an exemplary embodiment, the sizer 110 can be adjusted in the stomach 40 using a flexible and/or rigid grasper inserted into the stomach 40 through an abdominal access hole. The grasper can include an end effector having two opposed, movable jaws configured to grasp and move the sizer 110 once the sizer 110 has been adequately advanced into the patient 10 for the grasper to access it. A scoping device inserted into the stomach 40 can have a light located thereon which can help the surgeon find and grasp the sizer 110 with the grasper and to locate the pyloric valve. If the sizer 110 is advanced into the stomach 40 before the starting location 88 for the transection is determined and/or before the opening 96 is created at the starting location 88, the sizer's positioning along the lesser curvature 40c can assist in such determining and/or creating.

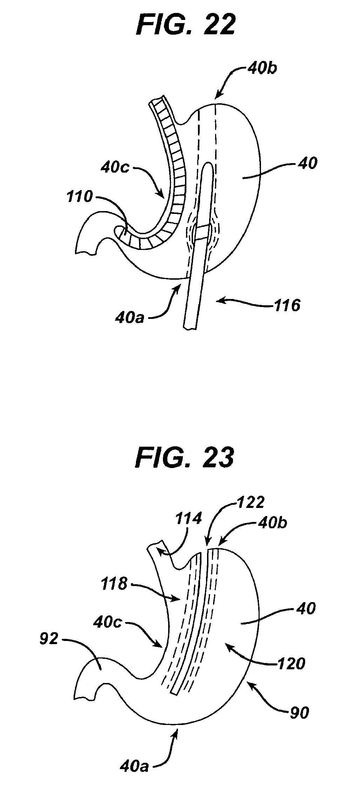

With the patient's stomach 40 prepared as desired, e.g., tissue attached to the stomach 40 dissected, stomach sleeve sized, transection starting location determined, etc., the stomach 164 can be transected between its lesser and greater curvatures 40c, 90. As discussed above, the stomach 40 can be transected in any way appreciated by a person skilled in the art, but in an exemplary embodiment a transector can be used to cut and secure the stomach 40 beginning at the opening 96. In one embodiment illustrated in FIG. 22, the stomach 40 can be transected using a transecting device 116 advanced into the patient 10 through one of the sealing ports 20a, 20b, 20c in the multiple port access device 16 positioned in the abdominal access hole 12, although the transector 116 can be inserted into the patient 10 in any way. The transection device 116 can be the same transector used to create the opening 96 to reduce the number of surgical instruments introduced to and removed from the patient 10, or as shown, a different transector, e.g., a linear surgical stapler, can be used. Non-limiting embodiments of a linear surgical stapler can be found in U.S. Patent No. 6,905,057 issued June 14, 2005 and entitled "Surgical Stapling Instrument Incorporating A Firing Mechanism Having A Linked Rack Transmission," U.S. Patent No. 7,111,769 issued September 26, 2006 and entitled "Surgical Instrument Incorporating An Articulation Mechanism Having Rotation About The Longitudinal Axis," U.S. Patent No. 6,786,382 issued September 7, 2004 and entitled "Surgical Stapling Instrument Incorporating An Articulation Joint For A Firing Bar Track," U.S. Patent No. 6,981,628 issued January 3, 2006 and entitled "Surgical Instrument With A Lateral-Moving Articulation Control," U.S. Patent No. 7,055,731 issued June 6, 2006 and entitled "Surgical Stapling Instrument Incorporating A Tapered Firing Bar For Increased Flexibility Around The Articulation Joint," U.S. Patent No. 6,964,363 issued November 15, 2005 and entitled "Surgical Stapling Instrument Having Articulation Joint Support Plates For Supporting A Firing Bar," U.S. Patent No. 6,959,852 issued November 1, 2005 and entitled "Surgical Stapling Instrument With Multistroke Firing Incorporating An Anti-Backup Mechanism," U.S. Patent Publication No. 2005/0070925 filed September 29, 2003 and entitled "Surgical Stapling Instrument Having Multistroke Firing With Opening Lockout," U.S. Patent No. 7,000,819 issued February 21, 2006 entitled "Surgical Stapling Instrument Having Multistroke Firing Incorporating A Traction-Biased Ratcheting Mechanism," and U.S. Patent No. 7,364,061 issued April 29, 2008 and entitled "Surgical Stapling Instrument Incorporating A Multistroke Firing Position Indicator And Retraction Mechanism," which are hereby incorporated by reference in their entireties.

In an exemplary embodiment, the transector 116 can be inserted through the opening 96, and it can be used to cut and secure the stomach 40 along a transection "line" 122 in a direction from the antrum 40a to the angle of His 40b, using the sizer 110 as a guide until the angle of His 40b is breached. The transection "line" 122 can generally include an opening in the stomach 40 that is closed or sealed using one or more securing elements, e.g., two rows of staples on either side of the opening as illustrated in FIG. 23. The stomach 40 can thereby be separated by the transection "line" 122 between the lesser curvature 40c and the greater curvature 90 to form a gastric tube or stomach sleeve 118 along the lesser curvature 40c that drains into the antrum 40a. Such a transection can separate the stomach fundus from an area of the stomach 40 substantially near the patient's esophagus 114 and allow the fundus to retain fluid communication with the patient's pylorus 92, and more specifically, with the patient's pyloric valve. During stomach transection, at least one grasper inserted through the multiple port access device 16 and/or other access hole in the patient 10 can be used to tension the stomach 40 and/or to hold the sizer 110 in a desired location along the stomach's lesser curvature 40c. The stomach could also or alternatively be tensioned by passing suture through a percutaneous opening, e.g., through a trocar or other port, and inserting the suture through the fundus of the stomach and back out the stomach and out the percutaneous port. The free ends of the suture can thus be tensioned to lift and stretch the stomach, thereby facilitating transaction.

The transection can be visualized using at least one scoping device inserted through any opening, as discussed herein. For non-limiting example only, the surgeon can visualize above and/or underneath the stomach 40 using, e.g., the laparoscope 38 inserted through the multiple port access device 16 in the abdominal access hole 12, to determine if a desired path of transection is clear or readily cleared of tissue and/or other debris. The surgeon can place one or more draining devices in the stomach fundus following the transection, e.g., along a greater curvature of the stomach sleeve formed by the transection. If used, the sizer 110 can be removed from the stomach 40 at any time during the surgical procedure, but in an exemplary embodiment it is removed from the patient 10 by retracting it through the patient's mouth 112 after the stomach 40 has been transected and inspected via scoping device visualization for any uncorrected and potentially dangerous irregularities, e.g., improperly bent staples, improperly placed staples, untied sutures, etc.

The surgeon can optionally secure the transected stomach, e.g., along the stapled or otherwise secured cut edge of the fundus, using any one or more supplemental securing elements in any combination to help better secure the transection and/or reduce bleeding. The supplemental securing elements are preferably biocompatible and can optionally be bioabsorbable such that the supplemental securing elements can dissolve in the patient 10 over time as the transection heals. Non-limiting embodiments of a surgical stapler than can apply staples with bioabsorbable pledgets can be found in previously filed U.S. Patent Application No. [Atty. Docket No. END5966], which is hereby incorporated by reference in its entirety.

At the conclusion of a gastroplasty, any access holes formed in a patient can be closed in any way and in any order as will be appreciated by a person skilled in the art, such as by suturing the openings.

The patient 10 can optionally be provided with a drug and/or device that suppresses appetite that can work in conjunction with the stomach sleeve to help the patient 10 lose weight. Such a drug or device can be provided to the patient 10 at the end of the gastroplasty and/or in a subsequent surgical procedure. A non-limiting embodiment of an implantable appetite suppressant device is available from Duocore, Inc. of Ramat-Hasharon, Israel.

A gastroplasty procedure described herein can optionally be combined with one or more other surgical procedures. For non-limiting example, the gastroplasty can be combined with a transoral minimally invasive surgical procedure, non-limiting examples of which, e.g., creating a gastroenteroanastomosis or enteroenteroanastomosis, can be found in U.S. Patent Application No. 2006/0271075 filed May 18, 2006 and entitled "Double Loop Gastric Bypass Procedure," which is hereby incorporated by reference in its entirety. As another non-limiting example, the gastroplasty can be performed as a first stage of a two stage surgical procedure where a second stage, e.g., a duodenal switch, a Roux-en-Y procedure, etc., can be performed immediately after the gastroplasty or in a subsequent surgical procedure.

A person skilled in the art will appreciate that the present invention has application in conventional endoscopic and open surgical instrumentation as well application in robotic-assisted surgery.

The devices disclosed herein can also be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical apparatus, comprising:
a housing having a plurality of sealing ports positionable in an abdominal wall of a patient; and
a surgical instrument adapted to be inserted through one of the plurality of sealing ports in the housing for transecting a greater curvature of a stomach of the patient from a lesser curvature of the stomach to form a gastric tube along the lesser curvature that drains into an antrum of the stomach.

2. The apparatus of claim 1, further comprising a scoping device adapted to be inserted through one of the plurality of sealing ports in the housing for visualizing an abdominal cavity of the patient.

3. The apparatus of claim 1, further comprising means for forming an opening through anterior and posterior walls of the stomach.

4. The apparatus of claim 2 and claim 3, wherein the scoping device is adapted to visualize the stomach between the antrum and an angle of His.

5. The apparatus of claim 1, further comprising a sizing device adapted to be transorally introduced into the stomach for sizing the portion of the stomach to be transected.

6. The apparatus of claim 1, further comprising a device adapted to be inserted through one of the plurality of sealing ports in the housing for retracting a liver of the patient.

7. The apparatus of claim 1, further comprising a grasper adapted to be inserted through one of the plurality of sealing ports in the housing for tensioning a tissue attached to the stomach, and a second surgical instrument adapted to be inserted through one of the plurality of sealing ports in the housing for detaching at least a portion of the tissue from the stomach.

8. The apparatus of claim 7, wherein the second surgical instrument includes an optically clear distal end to allow visualization of the stomach during detaching of at least a portion of the tissue.

9. The apparatus of claim 1, in which the surgical instrument further comprises at least one flexible joint along a longitudinal length of the surgical instrument to allow it to be positioned for transecting.

10. The apparatus of claim 1, further comprising a trocar for forming an access hole in the abdominal wall, and a grasper adapted to be inserted through the trocar for tensioning a tissue attached to the stomach.

11. The apparatus of claim 1, wherein the surgical instrument comprises a surgical stapler.

12. The apparatus of claim 1, wherein the housing is positionable to form an access hole through an umbilicus of the patient.

13. A surgical apparatus, comprising:
a housing having a plurality of sealing ports therein positionable in an abdominal wall of a patient;
a trocar for forming a second access hole in the abdominal wall; and
a surgical instrument adapted to be inserted through one of the housing and the trocar for transecting a portion of a stomach of the patient between an opening formed through posterior and anterior walls of the stomach and an angle of His of the stomach.

14. A surgical apparatus, comprising:
a housing having a plurality of sealing ports therein positionable in an abdominal wall of a patient;
a sizing device adapted to be transorally inserting into a stomach of the patient for sizing a portion of the stomach to be transected; and
a surgical stapler adapted to be inserted through the abdominal wall through one of the plurality of sealing ports in the housing for transecting the portion of the stomach.
